(19) **Europäisches Patentamt**

**European Patent Office**

**Office européen des brevets**

(11) **EP 1 379 313 B1**

(12) **FASCICULE DE BREVET EUROPEEN**

(45) Date de publication et mention
de la délivrance du brevet:
**15.02.2006 Bulletin 2006/07**

(51) Int Cl.:
***A61N 7/02*** *(2006.01)* ***G01R 33/48*** *(2006.01)*

(21) Numéro de dépôt: **02735460.4**

(22) Date de dépôt: **19.04.2002**

(86) Numéro de dépôt international:
**PCT/FR2002/001352**

(87) Numéro de publication internationale:
**WO 2002/085457 (31.10.2002 Gazette 2002/44)**

(54) **ENSEMBLE POUR LE TRAITEMENT THERMIQUE DE TISSUS BIOLOGIQUES**

SYSTEM ZUR THERMISCHEN BEHANDLUNG VON BIOLOGISCHEM GEWEBE

ASSEMBLY FOR HEAT TREATMENT OF BIOLOGICAL TISSUES

(84) Etats contractants désignés:
**AT BE CH CY DE DK ES FI FR GB GR IE IT LI LU
MC NL PT SE TR**

(30) Priorité: **20.04.2001 FR 0105410
22.08.2001 FR 0110992**

(43) Date de publication de la demande:
**14.01.2004 Bulletin 2004/03**

(73) Titulaires:
• **CENTRE NATIONAL DE
LA RECHERCHE SCIENTIFIQUE (CNRS)
75016 Paris (FR)**
• **Université Victor Segalen Bordeaux 2
33000 Bordeaux (FR)**

(72) Inventeurs:
• **MOONEN, Chrétien
F-33170 Gradignan (FR)**
• **QUESSON, Bruno
F-33000 Bordeaux (FR)**
• **VIMEUX, Frédéric
F-31700 Blagnac (FR)**

(74) Mandataire: **Callon de Lamarck, Jean-Robert et al
Cabinet Régimbeau
20, rue de Chazelles
75847 Paris cedex 17 (FR)**

(56) Documents cités:
**EP-A- 0 558 029 FR-A- 2 798 296
US-A- 5 368 031 US-A- 6 128 522**

**Description**

L'invention concerne les thérapies par hyperthermie locale.

Les thérapies par hyperthermie locale sont des techniques couramment utilisées pour traiter localement des tissus biologiques. Elles consistent à chauffer au moyen d'une source d'énergie (laser, micro-ondes, ondes radiofréquences, ultrasons, etc...) une zone cible du tissu biologique.

Ces techniques offrent de nombreux avantages. Du point de vue qualitatif, elles offrent un fort potentiel pour le contrôle de traitements tels que les thérapies géniques, le dépôt local de médicaments, l'ablation de tumeurs, etc. Du point de vue économique, elles sont compatibles avec un traitement ambulatoire des malades et permettent donc de réduire la durée d'hospitalisation.

D'une manière générale, les thérapies par hyperthermie locale permettent des interventions médicales dont la nature invasive est réduite au minimum.

Parmi les types d'énergie utilisés, les ultrasons focalisés (FUS) sont particulièrement intéressants puisqu'ils permettent de chauffer la zone de focalisation, de manière non invasive, profondément dans le corps biologique, sans chauffer de manière significative les tissus voisins de la zone focalisée.

Pendant le traitement, la température de la zone cible et de son environnement immédiat doit être contrôlée de manière précise et continue, alors que l'apport en énergie est localisé et rapide (de l'ordre de quelques secondes).

Le document US 5 368 031 décrit un ensemble pour le traitement thermique de tissus biologiques comprenant un système d'imagerie par résonance magnétique, le système d'imagerie incluant une station de travail et un écran pour visualiser l'image de la zone de tissus à traiter, une source d'énergie pulsée et un guide mécanique permettant à un chirurgien de positionner la source d'énergie à partir de la station de travail.

Le brevet FR 2 798 296 déposé le 13 septembre 1999 au nom du Centre National de la Recherche Scientifique (CNRS), décrit un ensemble pour le traitement thermique de tissus biologiques. L'ensemble décrit dans ce document prend en compte la distribution spatiale réelle de la température dans la zone cible et dans son environnement immédiat. Cette distribution spatiale permet d'estimer avec précision quelle doit être la quantité d'énergie à déposer et de commander la source d'énergie en conséquence. Un tel ensemble permet à la fois d'obtenir rapidement la température souhaitée dans la zone cible et de maintenir et contrôler la température dans cette zone cible avec une précision accrue, par rapport à ce qui était possible avec les techniques antérieures.

L'inconvénient de cet ensemble est qu'il est basé sur une modélisation d'une région chauffée très localisée dans l'espace. Par conséquent il permet le contrôle de l'évolution de la température dans la zone cible, mais ne permet pas un contrôle de la répartition des températures lorsque plusieurs sources d'énergie sont utilisées ou lorsque l'énergie est déposée simultanément en plusieurs endroits, par exemple à l'aide d'un réseau d'émetteurs.

Le but de la présente invention est de pallier ces inconvénients en proposant un ensemble de traitement thermique permettant un contrôle étendu de la température dans une région des tissus vivants et applicable sans limitations spatiales concernant le dépôt d'énergie.

A cet effet, l'invention propose un ensemble pour le traitement thermique conforme à la revendication 1.

Les moyens pour distribuer spatialement et temporellement la puissance déposée sont, par exemple, constitués par un transducteur ultrasonore dont on commande le déplacement dans l'espace. La région chauffée peut donc être plus étendue que la distribution initiale de la source d'énergie.

L'ensemble de traitement thermique conforme à la présente invention prend avantageusement en compte la distribution spatiale de la température en chaque point de la région. Contrairement aux ensembles de traitement thermique de l'art antérieur, cette caractéristique permet de contrôler la répartition de l'énergie dans toute la région traitée et non pas seulement l'énergie déposée en un point de focalisation. Il permet ainsi un contrôle tridimensionnel et en temps réel de l'évolution des températures dans le tissu biologique traité.

Avantageusement, l'unité de contrôle de l'ensemble de traitement thermique peut comprendre des moyens pour estimer les pertes d'énergie dans la région du tissu, à partir d'une estimation de la conduction thermique et de la distribution spatiale de la température dans la région et son environnement. Ceci permet de faire évoluer la répartition des températures dans les tissus traités thermiquement plus rapidement vers une répartition de consigne.

L'unité de contrôle comprend des moyens de traitement pour prendre en compte la conduction thermique en chaque point de la région.

En particulier, l'unité de contrôle peut comprendre des moyens pour mesurer la température en chaque point d'une pluralité de points échantillonnant la région et à intervalles de temps réguliers et pour en déduire une estimation de l'évolution de la température en fonction de la conduction thermique d'un point à l'autre de l'échantillonnage spatial.

Selon cette mise en oeuvre, l'image de la région du tissu biologique est décomposée en voxels et chaque voxel est associé à un point de la région. L'unité de traitement associe à chaque point une conductivité thermique et une température. Cette décomposition dite « point par point » permet avantageusement de contrôler l'évolution de la température dans toute la région du tissu biologique.

Avantageusement, les moyens générateurs d'énergie peuvent émettre des ultrasons focalisés. Les ultrasons focalisés permettent de fournir de la chaleur dans une zone localisée, de manière non invasive, même si cette zone est située profondément dans le corps humain ou l'animal.

Avantageusement, les moyens pour mesurer et enregistrer la distribution spatiale comprennent un appareil d'Imagerie par Résonance Magnétique (IRM). L'IRM permet une cartographie complète et non invasive des températures dans la zone à traiter, avec une bonne résolution spatiale (de l'ordre du millimètre) et une excellente précision (de l'ordre de 1°C). En outre, les données recueillies par IRM peuvent aisément être traitées numériquement.

Dans une mise en oeuvre de l'invention, l'amplitude *pw* de puissance à fournir en un point $\vec{r}$ à un instant $t + \Delta t$ est calculée par une relation du type:

$$ pw = \frac{Tp(\vec{r}, t+\Delta t) - FT^{-1}(T*(\vec{k},t)e^{-k^2 D\Delta t})}{FT^{-1}\left(\alpha \dfrac{1-e^{-k^2 D\Delta t}}{Dk^2} S*(\vec{k})\right)} $$

où $Tp(\vec{r}, t+\Delta t)$ est la température de consigne en ce point à l'instant $t + \Delta t$, $FT^{-1}$ est un transformée de Fourier inverse, $T*(\vec{k},t)$ est la transformée de Fourier de la température mesurée à l'instant $t$, $D$ est le coefficient de diffusion de la chaleur dans les tissus, $\alpha$ est le coefficient d'absorption de l'énergie des tissus, $S*(\vec{k})$ est la transformée de Fourier de la distribution spatiale de l'énergie déposée $S(\vec{r})$.

De cette manière, à chaque instant, l'énergie à déposer est contrôlée automatiquement par les moyens de traitement de manière à forcer la température à suivre un profil de consigne prédéfini. Cette caractéristique permet d'assurer une sécurité optimale pour le patient. Dans la pratique, ceci revient à calculer la puissance pw à appliquer entre deux mesures successives de température obtenues par IRM.

Bien entendu, les moyens générateurs d'énergie permettant d'induire une hyperthermie dans la région des tissus à traiter comprennent des sources d'énergie de type à ultrasons, laser, micro-ondes ou radiofréquence.

D'autres caractéristiques et avantages ressortiront encore de la description qui suit, laquelle est purement illustrative et non limitative et doit être lue en regard des figures annexées parmi lesquelles:

- la figure 1 représente schématiquement l'ensemble de traitement thermique conforme à l'invention,
- la figure 2 représente l'évolution de la température au point focal du transducteur en fonction du temps lorsque le procédé est appliqué à un gel acrylamide (échantillon test),
- la figure 3 représente l'évolution de la puissance des ultrasons focalisés en fonction du temps lorsque le procédé est appliqué à un gel acrylamide,
- la figure 4 représente l'évolution de la température au point focal du transducteur en fonction du temps lorsque le procédé est appliqué à un échantillon de viande fraîche,
- la figure 5 représente l'évolution de la puissance des ultrasons focalisés en fonction du temps lorsque le procédé est appliqué à un échantillon de viande fraîche,
- la figure 6 représente l'évolution de la température au point focal du transducteur en fonction du temps lorsque le procédé est appliqué *in vivo* à la cuisse d'un lapin,
- la figure 7 représente l'évolution de la puissance des ultrasons focalisés en fonction du temps lorsque le procédé est appliqué à la cuisse d'un lapin,
- la figure 8 représente la variation du minimum de la différence entre la température simulée et le profil de consigne comme une fonction de l'erreur sur les paramètres de diffusion et d'absorption des tissus traités calculée comme le rapport $\dfrac{(D/\alpha)\text{érroné}}{(D/\alpha)\text{optimal}}$,
- la figure 9 représente la variation de l'écart type de la différence entre la température simulée et le profil de consigne comme une fonction de l'erreur sur les paramètres de diffusion et d'absorption des tissus traités calculée comme

le rapport $\dfrac{\left(D/\alpha\right)\text{érroné}}{\left(D/\alpha\right)\text{optimal}}$.

Un des modes de réalisation de l'invention est décrit ci-dessous de manière détaillée. A titre d'exemple, ce mode de réalisation de l'invention correspond à un ensemble de traitement par hyperthermie locale par ultrasons focalisés (FUS), contrôlés par imagerie par résonance magnétique (IRM).

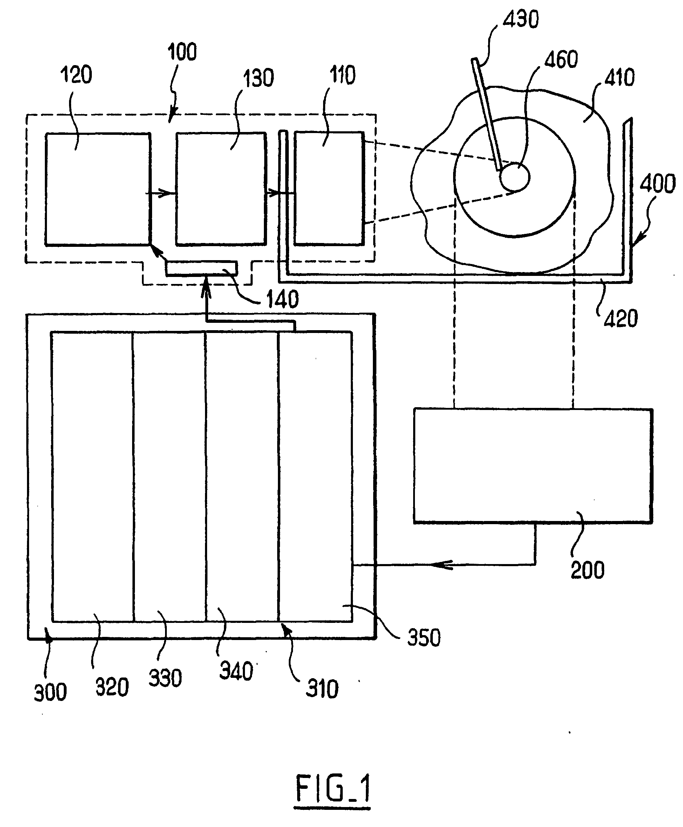

Comme représenté sur la figure 1, un tel ensemble comprend:

- des moyens générateurs d'ultrasons 100,
- des moyens de cartographie anatomique et de la température 200,
- une unité de contrôle de la température 300,
- un porte-échantillon 400 pour le tissu biologique 410 à traiter.

Dans le mode de réalisation de l'invention décrit ici, les moyens générateurs d'énergie 100 sont composés d'un transducteur 110 apte à être déplacé par un système hydraulique, d'un générateur de signal sinusoïdal 120, d'un amplificateur 130 et d'un convertisseur 140, reliant le générateur de signal sinusoïdal 120, à l'unité de contrôle 300.

Le transducteur 110 présente un diamètre de 90 mm avec un rayon de courbure de 80 mm. La distance focale peut être réglée électroniquement entre 50 et 125 mm et la position de la région focale peut être modifiée mécaniquement dans le plan horizontal dans une gamme de 80mmx80mm. Il opère à 1,5 MHz. Le signal d'entrée est généré par un générateur d'ondes carrées multi-canaux. Les signaux sont filtrés afin d'éviter les interférences avec les instruments à résonance magnétique opérant par exemple à 63 MHz pour un appareillage IRM 1,5T.

Le générateur 120 est, par exemple, un générateur mulitcanaux (société Corelec) piloté par une liaison série. Le système de déplacement du transducteur dans le plan horizontal est, par exemple, un système hydraulique piloté par une liaison série.

Les deux liaisons précitées sont connectées, par exemple, au PC recevant les images IRM en temps réel et réalisant les cartographies de température afin de permettre l'asservissement de température souhaitée.

Les moyens de cartographie 200 permettent de mesurer et d'enregistrer la distribution spatiale de la température. Ils comprennent, par exemple un appareil IRM de type ACS NT 1.5 T commercialisé par la société Philips ® (Best, Pays Bas). L'unité de contrôle 300 comprend en particulier une station de travail 310 de type PC, commercialisé par la société Dell®. Le PC permet de contrôler le générateur d'ultrasons 100 et le système de déplacement du transducteur 110. Dans ce dispositif, tous les paramètres de dépôt d'énergie par ultrasons focalisés sont donc réglables par l'intermédiaire de la station de travail: la puissance des ultrasons, la distance focale et la position du transducteur 110. La station de travail comprend en outre une interface graphique permettant de visualiser en temps réel l'état d'avancement de l'intervention.

L'unité de contrôle 300 comporte également des moyens d'évaluation et de traitement numérique de la distribution spatiale de la température 320, des moyens de détermination de la valeur de la puissance 330 devant être fournie à une zone cible de la région contrôlée, des moyens 340 d'estimation des pertes d'énergie thermique dans la région considérée et des moyens de commande 350 des moyens générateurs d'énergie. Les moyens de commande 350 indiquent aux moyens générateurs d'énergie 100 de délivrer la valeur de la puissance fournie par les moyens de détermination de la valeur de la puissance 330.

Le porte-échantillon 400 comprend un support 420. Ce support contient le transducteur 110 et une bobine de surface (récepteur de signaux IRM). Le support 420 est placé dans un réservoir rempli d'eau afin d'assurer une propagation optimale des ultrasons focalisés vers les tissus cibles. L'eau est maintenue à une température constante de 38°C en utilisant un contrôleur de température d'eau de bain (par exemple Polysciences, model 9110-BB,IL, Etats-Unis) pour éviter le refroidissement des échantillons testés.

Le but d'un procédé automatique de contrôle de température est de forcer la température à une position donnée de la région des tissus à traiter à suivre un profil de consigne Tp(t). L'évolution de la température dans l'espace et dans le temps est donnée par l'équation bio-chaleur [1] qui prend en compte les coefficients d'absorption d'énergie par les tissus ($\alpha$) et de diffusion de la chaleur (D) dans les tissus:

$$\frac{\partial T(\vec{r},t)}{\partial t} = D.\nabla^2 T(\vec{r},t) + \alpha\, S(\vec{r}).pw(t) \qquad [1]$$

où $T(\vec{r}, t)$ est la cartographie des températures, $\nabla^2$ est l'opérateur Laplacien, $S(\vec{r})$ est la distribution spatiale de l'énergie déposée et pw(t) est son amplitude.

Cette équation ne prend pas en compte la perfusion tissulaire ou la chaleur produite par le métabolisme car la chaleur générée est négligeable devant la quantité de chaleur déposée par ultrasons focalisés (FUS). L'invention généralise le principe de contrôle à base de l'équation 1 sans contrainte concernant la distribution spatiale du dépôt d'énergie en prenant en compte le transport de chaleur de chaque point (ou voxel) vers chaque autre point (ou voxel). Pour cela, on recherche une solution analytique de l'équation [1], afin de prédire au mieux l'évolution temporelle de la température en tout point de l'espace, en fonction de la diffusion et du dépôt d'énergie par la source. La transformation de Fourier sur les coordonnées spatiales de l'équation [1] conduit à une équation linéaire du premier ordre en fonction du temps:

$$\frac{\partial T^*(\vec{k},t)}{\partial t} = -k^2 D T^*(\vec{k},t) + \alpha\, S^*(\vec{k}).pw(t) \qquad [2]$$

où $T^*(\vec{k}, t)$t) et $S^*(\vec{k})$ sont les transformées de Fourier sur les coordonnées spatiales de $T(\vec{r}, t)$ et $S(\vec{r})$ respectivement.

Une solution peut être dérivée de l'équation [2] en supposant que la puissance pw(t) est constante pendant un intervalle de temps $\Delta t$ donné (correspondant à l'intervalle de mesure de température par IRM):

$$T^*(\vec{k},t+\Delta t) = e^{-k^2 D\Delta t}T^*(\vec{k},t) + \alpha\frac{1-e^{-k^2 D\Delta t}}{Dk^2}S^*(\vec{k}).pw \qquad [3]$$

Par conséquent, la puissance à appliquer durant $\Delta t$ pour forcer la température $T(\vec{r}, t+\Delta t)$ à être égale à un profil de température $Tp(\vec{r}, t+\Delta t)$ peut être dérivée de la transformation de Fourier inverse (FT$^{-1}$) de l'équation [3]:

$$pw = \frac{Tp(\vec{r},t+\Delta t) - FT^{-1}(T^*(\vec{k},t)e^{-k^2 D\Delta t})}{FT^{-1}\left(\alpha\frac{1-e^{-k^2 D\Delta t}}{Dk^2}S^*(\vec{k})\right)} \qquad [4]$$

Ce type d'algorithme permet d'assurer une sécurité optimale pour le patient puisqu'il permet de contrôler de manière automatisée la température. A cet effet, l'énergie à déposer pour forcer la température à suivre un profil de consigne prédéfini est évaluée à intervalles de temps $\Delta t$ réguliers. Dans la pratique, ceci revient à calculer la puissance pw à appliquer entre deux mesures successives de température obtenues par IRM. D'une manière idéale, ce type d'algorithme prend en compte le phénomène physique (ici l'équation de diffusion de la chaleur) et est le plus robuste possible.

## _Réglage du dispositif_

Toutes les expériences ont été réalisées en suivant le même protocole. On acquiert la position d'un volume de référence pour définir une région d'intérêt et la position du point focal de référence. On relève la position du volume de référence par rapport à l'isocentre de l'aimant de l'appareil IRM pour positionner le transducteur 110 et ajuster la distance focale. Ensuite, on réalise un scan répétitif de ce volume pour préparer le processus de chauffage. Cette préparation est utilisée pour:

-   calculer l'écart standard à la moyenne de température en chaque voxel du volume afin d'estimer la précision de la mesure de la température,

- corriger la position du transducteur 110 et sa distance focale: on applique des ultrasons focalisés de faible puissance durant un court instant (de l'ordre de 5 s) afin d'induire une hyperthermie modérée (environs + 3°C). Cette mesure permet de vérifier les coordonnées de position de l'image par résonance magnétique et on ajuste la position du transducteur et la distance focale si nécessaire,
- évaluer les paramètres de diffusion D et d'absorption α du tissu: on applique des ultrasons focalisés durant un court instant et on réalise un ajustement non-linéaire par la méthode des moindres carrés sur la courbe de l'évolution de température au point focal en fonction du temps afin d'obtenir ces paramètres.

Après ce protocole préparatoire, on programme l'évolution désirée de température en fonction du temps (profil de consigne $Tp(\vec{r},t)$) et on entame le processus de contrôle automatique (équation 4).

Pour permettre à ce processus de fonctionner correctement, il est nécessaire de réaliser une synchronisation entre l'acquisition par IRM et le PC pilotant les ultrasons focalisés. A cet effet, le dispositif d'imagerie IRM génère un signal TTL (Time to Live selon la terminologie anglo-saxonne couramment utilisée) au début de chaque scan. Ce signal est détecté par une interface intégrée qui commute un relais connecté à un port parallèle du PC. On détecte cette commutation grâce à une routine spécifique en langage C et on enregistre les temps systèmes du PC correspondant dans un module de mémoire partagé utilisé par l'algorithme. Les délais ainsi mesurés sont pris en compte dans l'algorithme de contrôle de température.

*Expérimentation*

Des expériences sur des gels fantômes, des échantillons de viande fraîche et des cuisses de lapin *in vivo* ont été réalisées sur le système ACS/NT Philips 1,5 Tesla équipé du prototype Philips de génération d'ultrasons focalisés permettant d'induire une hyperthermie locale. Des lapins ont été anesthésiés et positionnés de telle manière que les muscles des cuisses soient centrés approximativement sur le faisceau d'ultrasons. Les valeurs des coefficients α et D issues des mesures préliminaires sont présentées dans le tableau ci-dessous :

| | Gel acrylamide | Viande fraîche | Cuisse de lapin (*in vivo*) |
|---|---|---|---|
| D (mm$^2$.s$^{-1}$) | 0,17 | 0,36 | 0,10 |
| α (°C.s$^{-1}$.%$^{-1}$) | 0,33 | 0,29 | 0,40 |

Lorsque la phase préparatoire d'ajustement a été réalisée (voir précédemment), le protocole de contrôle de température en temps réel est effectué.

Dans ces expériences, une résolution temporelle de 1,75 secondes pour 3 coupes parallèles a été obtenue, en utilisant une séquence d'imagerie « EPI segmentée » avec les paramètres suivants: un temps d'écho (TE) de 30 ms, un temps de répétition (TR) de 60ms et 11 étapes d'encodage de phase par TR avec une résolution spatiale de 1 x 1 mm, 3 mm épaisseur de coupe.

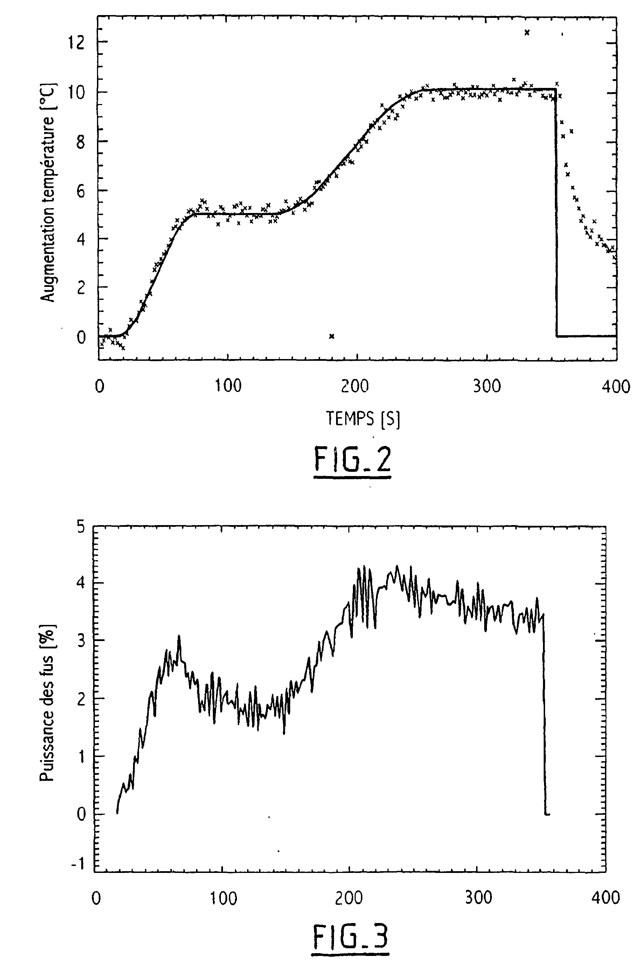

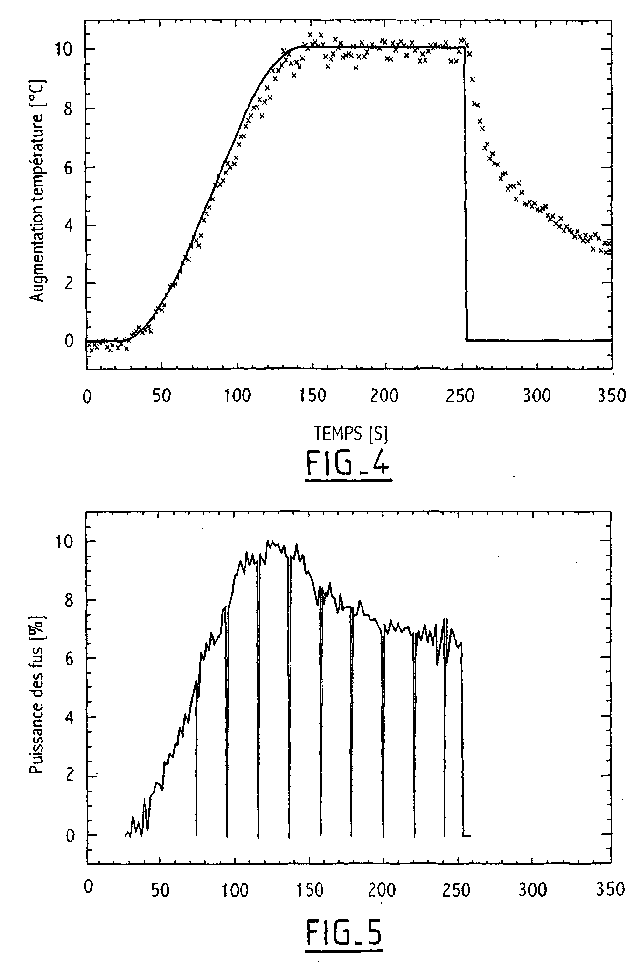

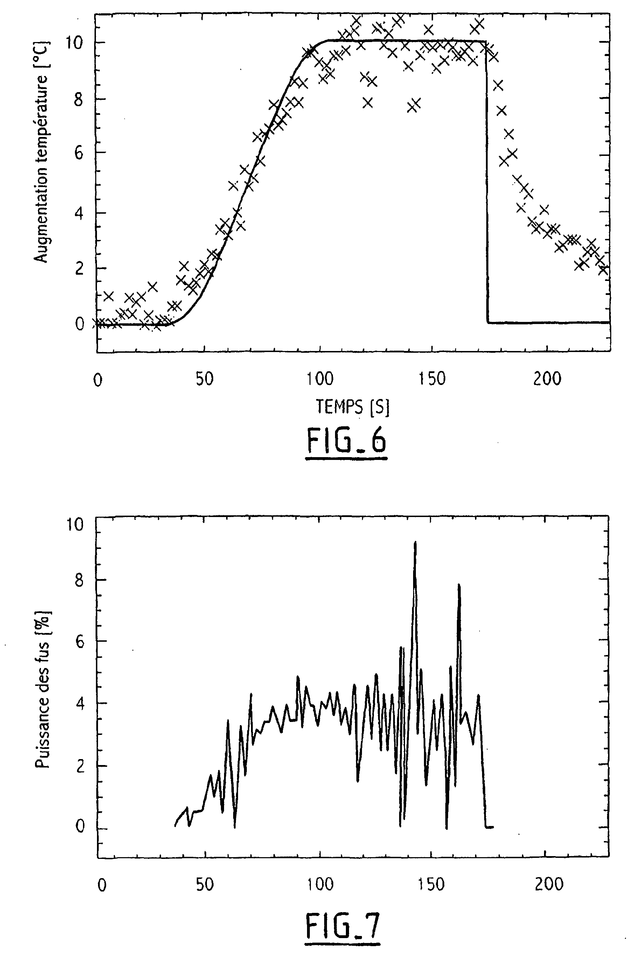

Les figures 2, 4 et 6 représentent l'évolution de la température au point focal du transducteur d'ultrasons focalisés en fonction du temps obtenue respectivement sur le gel acrylamide, sur un échantillon de viande fraîche et sur une cuisse de lapin. La courbe en ligne continue représente le profil de température de consigne Tp(t) et les symboles représentent les données expérimentales de température au point focal, mesurées par IRM de température. Comme on le voit sur la figure 6, l'application des ultrasons focalisés a été stoppée au bout de 170 s. La température a alors décru vers sa valeur initiale, sans aucun contrôle, à cause du phénomène de diffusion.

L'écart type de la différence entre la température mesurée et la température de consigne est resté relativement constant (0,75°C en moyenne) pendant la phase d'hyperthermie, indiquant que la méthode proposée permet de réaliser un contrôle efficace en temps réel de l'évolution de la température *in vivo*.

Les figures 3, 5 et 7 représentent l'évolution de la puissance des ultrasons focalisés en fonction du temps lorsque le procédé est appliqué respectivement au gel acrylamide, à l'échantillon de viande fraîche et à la cuisse de lapin.

Il est évident que les valeurs des coefficients α et D peuvent varier au cours de l'expérience (en fonction de la température, à cause d'une dénaturation des protéines, d'un changement de perfusion, etc...). Il est donc important de s'assurer que l'algorithme de contrôle de température proposé n'est pas très sensible à une variation de ces paramètres.

La sensibilité de la qualité du contrôle de température a été estimée à partir de simulations numériques, en faisant varier les paramètres D et α sur une large gamme de valeurs, respectivement entre 30% et 230%, et entre 50% et 150% de leur valeur estimée initialement (à partir de la phase préparatoire) avec un pas de 2%. Pour chaque couple

($\alpha$ ,D) l'évolution de température a été calculée en utilisant la puissance appliquée réellement pendant l'expérience. Les résultats obtenus montrent que la température suit le profil de température avec un décalage et une oscillation plus ou moins importants. Le minimum de la différence entre la température simulée et la température de consigne donne la valeur de décalage et l'écart type de cette différence permet l'évaluation de l'amplitude de l'oscillation.

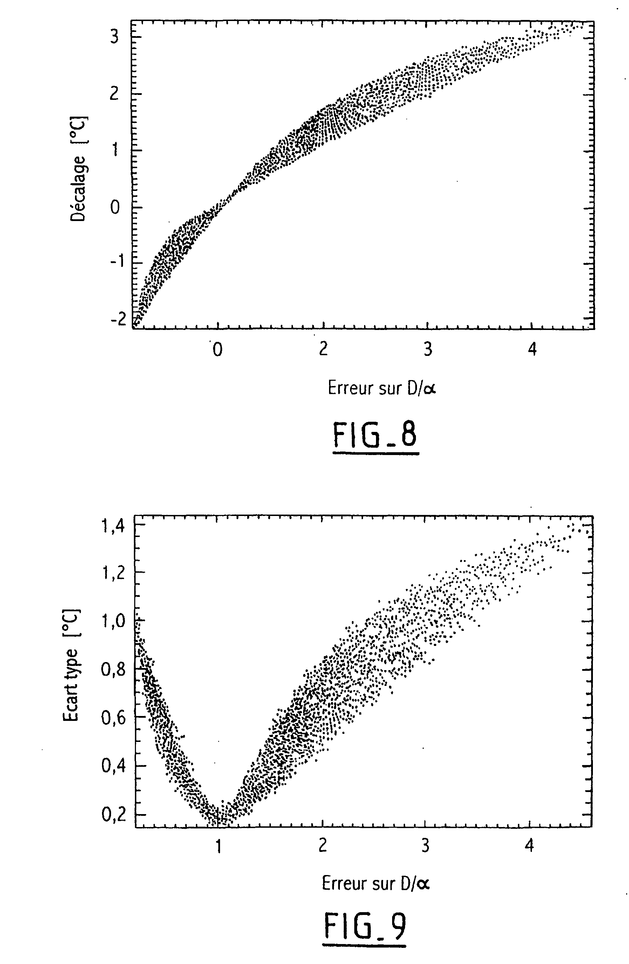

La figure 8 représente la variation du minimum de la différence entre la température simulée et le profil de consigne comme une fonction de l'erreur sur les paramètres de diffusion et d'absorption des tissus traités calculée

comme le rapport $\dfrac{\left(D/\alpha\right)\text{érroné}}{\left(D/\alpha\right)\text{optimal}}$.

La figure 9 représente la variation de l'écart type de la différence entre la température simulée et le profil de consigne comme une fonction de l'erreur sur les paramètres de diffusion et d'absorption des tissus traités calculée

comme le rapport $\dfrac{\left(D/\alpha\right)\text{érroné}}{\left(D/\alpha\right)\text{optimal}}$.

Ces résultats font apparaître une corrélation importante entre l'erreur sur D/$\alpha$ et la précision de l'algorithme de contrôle. De plus, il apparaît qu'une erreur d'estimation sur $\alpha$ et D (due notamment à leur variation au cours de l'expérience) altère peu la qualité du contrôle. Ces résultats confirment l'efficacité et la robustesse de la méthode proposée.

Le contrôle de la température en temps réel de l'hyperthermie locale peut être réalisé *in vivo* sur un IRM clinique. Cette méthode simple et prédictive basée sur le modèle physique de la diffusion de la température dépend uniquement des coefficients d'absorption ($\alpha$) et de diffusion (D) des tissus. L'expression mathématique de l'algorithme proposé est très générale et par conséquent applicable à n'importe quelle source d'énergie (Ultrasons focalisés, Radiofréquence, Laser, Micro-ondes, etc...) permettant d'induire une hyperthermie des tissus biologiques. La seule condition requise à sa mise en oeuvre est la connaissance du profil spatial du dépôt d'énergie.

## Revendications

1. Ensemble pour le traitement thermique d'une région d'un tissu biologique (410) comprenant:

    - des moyens générateurs d'énergie (100) pour fournir de l'énergie dans la région,
    - des moyens (200) pour mesurer et enregistrer la distribution spatiale de température dans ladite région,
    - une unité de contrôle (300) comprenant des moyens de traitement numérique point par point de la distribution spatiale de température dans la région,

    **caractérisé en ce que** les moyens générateurs d'énergie comportent des moyens (110) pour distribuer spatialement et temporellement la puissance qu'ils déposent sur la région précitée, l'unité de contrôle (300) comportant des moyens automatiques (330, 350) pour, en fonction de la distribution de température et de la conduction thermique en chaque point de la région, commander la quantité et la répartition de l'énergie déposée.

2. Ensemble de traitement thermique selon la revendication 1, **caractérisé par le fait que** l'unité de contrôle (300) comprend en outre des moyens (340) pour estimer les pertes d'énergie dans la région du tissu (410), à partir d'une estimation de la conduction thermique et de la distribution spatiale de la température dans la région et son environnement.

3. Ensemble de traitement thermique selon la revendication 1 ou 2, **caractérisé en ce que** l'unité de contrôle (300) comprend des moyens (320) pour mesurer la température en chaque point d'une pluralité de points échantillonnant la région et à intervalles de temps réguliers et pour en déduire une estimation de l'évolution de la température en fonction de la conduction thermique d'un point à l'autre de l'échantillonnage spatial.

4. Ensemble de traitement thermique selon l'une des revendications précédentes, **caractérisé par le fait que** les moyens générateurs d'énergie (100) émettent des ultrasons focalisés.

5. Ensemble de traitement thermique selon l'une des revendications précédentes, **caractérisé par le fait que** les moyens (200) pour mesurer et enregistrer la distribution spatiale de température comprennent un appareil d'imagerie par résonance magnétique.

**6.** Ensemble de traitement thermique selon l'une des revendications précédentes, **caractérisé en ce que** l'amplitude *pw* de puissance à fournir en un point F à un instant *t* + Δ*t* est calculée par une relation du type:

$$pw = \frac{Tp(\vec{r}, t + \Delta t) - FT^{-1}(T * (\vec{k}, t) e^{-k^2 D \Delta t})}{FT^{-1}\left( \alpha \frac{1 - e^{-k^2 D \Delta t}}{Dk^2} S * (\vec{k}) \right)}$$

où $Tp(\vec{r}, t + \Delta t)$ est la température de consigne en ce point à l'instant *t* + Δ*t*, $FT^{-1}$ est un transformée de Fourier inverse, $T * (\vec{k}, t)$ est la transformée de Fourier de la température mesurée à l'instant *t*, *D* est le coefficient de diffusion de la chaleur dans les tissus, a est le coefficient d'absorption de l'énergie des tissus, $S * (\vec{k})$ est la transformée de Fourier de la distribution spatiale de l'énergie déposée $S(\vec{r})$.

**7.** Ensemble de traitement thermique selon l'une des revendications précédentes, **caractérisé par le fait que** les moyens générateurs d'énergie (100) comprennent des sources d'énergie de type à ultrasons, laser, micro-ondes ou radiofréquence.

**Patentansprüche**

**1.** Anordnung für die thermische Behandlung eines Bereiches eines biologischen Gewebes (410), mit

- Energieerzeugungsmitteln (100) zum Liefern der Energie in den Bereich,
- Mitteln (200) zum Messen und Aufnehmen der räumlichen Temperaturverteilung in dem Bereich,
- einer Steuereinheit (300) mit Mitteln zur digitalen Punkt-für-Punkt-Bearbeitung der räumlichen Temperaturverteilung in dem Bereich,

**dadurch gekennzeichnet, daß** die Energieerzeugungsmittel Mittel (110) zum räumlichen und zeitlichen Verteilen der Leistung aufweisen, die sie in dem vorgenannten Bereich deponieren, wobei die Steuereinheit (300) automatische Mittel (330, 350) zum Steuern, abhängig von der Temperaturverteilung und der Wärmeleitung in jedem Punkt des Bereiches, der Menge und der Verteilung der deponierten Energie umfaßt.

**2.** Wärmebehandlungsanordnung nach Anspruch 1, **dadurch gekennzeichnet, daß** die Steuereinheit (300) außerdem Mittel (340) zum Schätzen der Energieverluste in dem Bereich des Gewebes (410) ausgehend von einer Schätzung der Wärmeleitung und der räumlichen Temperaturverteilung in dem Bereich und seiner Umgebung umfaßt.

**3.** Wärmebehandlungsanordnung nach Anspruch 1 oder 2, **dadurch gekennzeichnet, daß** die Steuereinheit (300) Mittel (320) zum Messen der Temperatur an jedem Punkt einer Vielzahl von den Bereich abtastenden Punkten und in gleichmäßigen Zeitintervallen und zum daraus Ableiten einer Schätzung der Temperaturentwicklung abhängig von der Wärmeleitung von einem Punkt zu einem anderen der räumlichen Abtastung umfaßt.

**4.** Wärmebehandlungsanordnung nach einem der vorstehenden Ansprüche, **dadurch gekennzeichnet, daß** die Energieerzeugungsmittel (100) fokussierte Ultraschallwellen emittieren.

**5.** Wärmebehandlungsanordnung nach einem der vorstehenden Ansprüche, **dadurch gekennzeichnet, daß** die Mittel (200) zum Messen und Aufnehmen der räumlichen Temperaturverteilung eine Magnetresonanzbildgebungsvorrichtung umfassen.

**6.** Wärmebehandlungsanordnung nach einem der vorstehenden Ansprüche, **dadurch gekennzeichnet, daß** die Amplitude pw der an einen Punkt $\bar{r}$ zu einem Zeitpunkt $t+\Delta t$ zu liefernden Leistung mittels einer Gleichung vom Typ berechnet wird:

$$ pw = \frac{Tp(\bar{r}, t + \Delta t) - FT^{-1}(T*(\bar{k}, t)e^{-k^2 D\Delta t})}{FT^{-1}\left( \alpha \frac{1 - e^{-k^2 D\Delta t}}{Dk^2} S*(\bar{k}) \right)} $$

wobei $Tp(\bar{r}, t + \Delta t)$ die Solltemperatur an diesem Punkt zum Zeitpunkt $t+\Delta t$ *ist, FT$^{-1}$* eine inverse Fourier-Transformierte ist, $T*(\bar{k}, t)$ die Fourier-Transformierte der gemessenen Temperatur zum Zeitpunkt $t$ ist, $D$ der Wärmediffusionskoeffizient in den Geweben ist, $\alpha$ der Energieabsorptionskoeffizient der Gewebe ist, $S*(\bar{k})$ die Fourier-Transformierte der räumlichen Verteilung der deponierten Energie $S(\bar{r})$ ist.

**7.** Wärmebehandlungsanordnung nach einem der vorstehenden Ansprüche, **dadurch gekennzeichnet, daß** die Energieerzeugungsmittel (100) Ultraschall-, Laser-, Mikrowellen- oder Funkwellen-Energiequellen umfassen.

**Claims**

**1.** Assembly for the heat treatment of a region of biological tissue (410) comprising:

- energy-generating means (100) to supply energy to the region,
- means (200) for measuring and recording the spatial temperature distribution in said region,
- a control unit (300) comprising numerical processing means for a point-by-point processing of the spatial temperature distribution in the region,

**characterized in that** the energy-generating means comprise means (110) for spatially and temporally distributing the power that they apply to the aforesaid region, the control unit (300) comprising automatic means (330, 350) for, on the basis of the temperature distribution and of the thermal conductivity at each point in the region, controlling the amount and distribution of the applied energy.

**2.** Heat treatment assembly according to Claim 1, **characterized in that** the control unit (300) further comprises means (340) for estimating the energy losses in the region of the tissue (410) on the basis of an estimate of the thermal conductivity and of the spatial temperature distribution in the region and its surroundings.

**3.** Heat treatment assembly according to Claim 1 or 2, **characterized in that** the control unit (300) comprises means (320) for measuring the temperature at each point of a plurality of points sampling the region and at regular time intervals and for deducing therefrom an estimate of the change in temperature as a function of thermal conductivity from one point of the spatial sample to another.

**4.** Heat treatment assembly according to one of the preceding claims, **characterized in that** the energy-generating means (100) emit focused ultrasound.

**5.** Heat treatment assembly according to one of the preceding claims, **characterized in that** the means (200) for measuring and recording the spatial temperature distribution comprise a magnetic resonance imaging apparatus.

**6.** Heat treatment assembly according to one of the preceding claims, **characterized in that** the amplitude pw of the power to be supplied at a point $\bar{r}$ at an instant $t+\Delta t$ is calculated using a relationship of the type:

$$pw = \frac{Tp(\vec{r} + \Delta t) - FT^{-1}(T^*(\vec{k},t)e^{-k^2 D\Delta t})}{FT^{-1}\left(\alpha \frac{1 - e^{-k^2 D\Delta t}}{Dk^2} S^*(\vec{k})\right)}$$

where $Tp(\vec{r}, t+\Delta t)$ is the reference temperature at that point at the moment t+∆t, $FT^{-1}$ is an inverse Fourier transform, $T^*(\vec{k}, t)$ is the Fourier transform of the temperature measured at the moment t, D is the heat diffusion coefficient for the tissue, $\alpha$ is the energy absorption coefficient for the tissue, $S^*(\vec{k})$ is the Fourier transform of the spatial distribution of the applied energy $S(\vec{r})$.

7. Heat treatment assembly according to one of the preceding claims, **characterized in that** the energy-generating means (100) comprise energy sources of the ultrasound, laser, microwave or radiofrequency type.

FIG_1

FIG.2

FIG.3

FIG_4

FIG_5

FIG.6

FIG.7

14

FIG_8

FIG_9